## Europäisches Patentamt
## European Patent Office
## Office européen des brevets

(11) Veröffentlichungsnummer: **0 092 712**
**B1**

(12) **EUROPÄISCHE PATENTSCHRIFT**

(45) Veröffentlichungstag der Patentschrift:
24.02.88

(21) Anmeldenummer: **83103396.4**

(22) Anmeldetag: **07.04.83**

(51) Int. Cl.⁴: **A 61 M 5/14**

(54) **Druckinfusionsapparat für medizinische Anwendungen.**

(30) Priorität: **28.04.82 DE 3215711**

(43) Veröffentlichungstag der Anmeldung:
**02.11.83 Patentblatt 83/44**

(45) Bekanntmachung des Hinweises auf die Patenterteilung:
**24.02.88 Patentblatt 88/8**

(84) Benannte Vertragsstaaten:
**AT BE CH DE FR GB IT LI LU NL SE**

(56) Entgegenhaltungen:
**EP-A-0 042 282**
**DE-A-2 343 207**
**DE-A-3 144 825**
**GB-A-1 528 385**
**US-A-3 886 938**

(73) Patentinhaber: **INTERMEDICAT GMBH,**
**Gerliswilstrasse 74, CH- 6020 Emmenbrücke (CH)**

(72) Erfinder: **Mardorf, Robert, Zur Schlade 29, D-3508**
**Melsungen (DE)**

(74) Vertreter: **von Kreisler, Alek, Dipl.- Chem.,**
**Deichmannhaus am Hauptbahnhof, D-5000 Köln 1**
**(DE)**

EP 0 092 712 B1

LIBER, STOCKHOLM 1988

**Beschreibung**

Der Erfindung betrifft einen Druckfusionsapparat für medizinische Anwendungen.

Derartige Druckinfusionsapparate dienen dazu, einem Patienten mit einer Spritze ein flüssiges Medikament über einen längeren Zeitraum hinweg gleichmäßig verteilt zuzuführen, indem der Spritzeninhalt langsam aus der Spritze ausgedrückt wird. Die bekannten Druckinfusionsapparate weisen demnach eine Abstützvorrichtung zum Abstützen des Spritzenzylinders auf, während auf die Kolbenstange ein Antriebsorgan einwirkt, das sich entlang eines linearen Weges bewegt und die Kolbenstange vorschiebt.

Die in Verbindung mit den genannten Druckinfusionsapparaten verwendeten Spritzen sind im allgemeinen Einmalspritzen aus Kunststoff, die nach einmaliger Verwendung fortgeworfen werden. Diese Spritzen sind so ausgebildet, daß an dem Spritzenzylinder ein Abstützelement und an der Kolbenstange das Antriebsorgan angreifen kann.

Aus GB-A-1 528 385 ist ein Druckinfusionsapparat bekannt, bei dem eine Spindelmutter vorgesehen ist, die gegen das rückwärtige Ende der Kolbenstange der Spritze drückt. Die Spindelmutter wird von einer rotierenden Spindel angetrieben und linear in Richtung auf die Spritze bewegt, während sie in einer Gleitführung gegen Drehung festgehalten wird. Die Spindel ragt in das hohle Innere der Kolbenstange hinein. Wenn die Spritze gefüllt ist, steht die Kolbenstange weit nach hinten vor. Um die Spindel in diesem Zustand in das Innere der Kolbenstange einführen zu können, ist die Spindel mit einer Gelenkkugel in dem Gehäuse des Druckinfusionsapparates gelagert. Dennoch ist das Einsetzen einer gefüllten Spritze in das Gehäuse schwierig. Bei Druckinfusionsapparaten, die in der Medizintechnik verwendet werden ist eine einfache Handhabung äußerst wichtig. Das Krankenhauspersonal muß imstande sein, eine neue Spritze in den Druckinfusionsapparat mit wenigen Handgriffen einzuführen.

Bekannt ist ferner ein Druckinfusionsapparat (EP 0 042 282), bei dem ein mit Zähnen versehenes Antriebsrad an der runden Kolbenstange angreift, um diese anzutreiben. Die Kolbenstange selbst ist nicht profiliert. Bei Überlastung besteht die Gefahr, daß das Antriebsrad die Kolbenstange ausfräst, womit die Infusion zum Stillstand kommt.

Der Erfindung liegt die Aufgabe zugrunde, einen Druckinfusionsapparat der eingangs genannten Art zu schaffen, dessen Antriebssystem vereinfacht ist und bei dem das Auswechseln der Spritze auf einfache Weise durchführbar ist.

Die Lösung dieser Aufgabe erfolgt erfindungsgemäß mit den Merkmalen des Patentanspruchs 1.

Nach der Erfindung wird die Linearbewegung, die erforderlich ist, um die Kolbenstange relativ zu dem Spritzenzylinder zu bewegen, nicht im Innern der Haltevorrichtung erzeugt, sondern erst unmittelbar an der Spritze. Der Linearantrieb und die Linearführung sind somit von der Haltevorrichtung auf die Spritze verlegt. Hierdurch ist es möglich, die Haltevorrichtung einschließlich der Antriebseinrichtung einfacher auszubilden und kostengünstiger herzustellen. Durch die an der Spritze erforderliche Anbringung einer Verzahnung oder eines Gewindes erhöhen sich die Kosten für die Herstellung der Spritze nicht, da Einmalspritzen aus Kunststoff hergestellt werden. Die integrale Anformung der Profilierung in Form einer Verzahnung oder in Form eines Gewindes ist ohne Schwierigkeiten und ohne wesentliche Kosten möglich.

Da nach der Erfindung die Umsetzung der Drehbewegung des Antriebs in eine Linearbewegung erst unmittelbar an der Spritze erfolgt, wird an der Haltevorrichtung nicht nur ein linearbeweglicher Schieber eingespart, der bei den bekannten Haltevorrichtungen über den gesamten Weg des Kolbenhubes der Spritze geführt sein muß, sondern man benötigt auch keine Führungsvorrichtung für den Schieber.

Wichtig ist, daß das rotierende Antriebsorgan eine relative Verschiebung der Kolbenstange zu dem Spritzenzylinder hervorruft. Dabei ist es prinzipiell ohne Bedeutung, ob der Spritzenzylinder festgehalten und die Kolbenstange bewegt wird oder ob die Kolbenstange festgehalten und der Spritzenzylinder bewegt wird. Wichtig ist nur, daß das jeweils in Bezug auf die Haltevorrichtung bewegbare Teil eine Profilierung aufweist, in die das rotierende Antriebsorgan eingreift. Normalerweise wird man den Spritzenzylinder festlegen und die Kolbenstange antreiben.

Gemäß einer bevorzugten Ausführungsform der Erfindung ist das Antriebsorgan ein Zahnrad, dessen Zähne in eine zahnstangenartige Verzahnung der Kolbenstange oder des Spritzenzylinders eingreifen. Diese Ausführungsform hat den Vorteil, daß die Verzahnung des bewegbaren Teiles in jeder Stellung dieses Teiles in die Verzahnung des Zahnrades eingesetzt werden kann, indem das bewegbare Teil (Spritzenzylinder oder Kolbenstange) radial an das Zahnrad herangeführt wird.

Bei einer anderen Ausführungsform der Erfindung weist das Antriebsorgan ein Gewinde auf, das mit einem Gegengewinde oder einer Verzahnung der Kolbenstange oder des Spritzenzylinders zusammenwirkt. Auf diese Weise kann ein Schnekkentrieb realisiert werden, wobei die relativ schnelle Drehung des Antriebsorgans in einen relativ langsamen Vorschub des bewegbaren Teiles der Spritze umgesetzt wird.

Zweckmäßigerweise ist ein den Spritzenzylinder in axialer Richtung festlegender Halter vorgesehen und das rotierende Antriebsorgan bewegt die Kolbenstange linear. Hierbei ist es vorteilhaft, den Spritzenzylinder auch in seinem rückwärtigen Bereich durch eine Stützvorrichtung abzustützen. Die Kolbenstange ist dann durch den Spritzenzylinder in axialer Richtung geführt. Andererseits ist es auch möglich, die Kolbenstange unterhalb des Antriebsorgans abzustützen, um gleichzeitig einen sicheren Eingriff der Profilierung der Kolbenstange in die Profilierung des Antriebsorgans zu gewährleisten.

Im folgenden werden unter Bezugnahme auf die Zeichnungen Ausführungsbeispiele der Erfindung näher erläutert.

Es zeigen:

Fig. 1 eine schematische Darstellung eines Druckinfusionsapparates, bei dem der Spritzenzylinder festgehalten und die Kolbenstange bewegt wird und

Fig. 2 eine schematische Darstellung eines Druckinfusionsapparates, bei dem die Kolbenstange festgehalten und der Spritzenzylinder bewegt wird,

Fig. 3 eine Seitenansicht eines Druckinfusionsapparates, der von einer an der Haltevorrichtung gelagerten Schnecke angetrieben wird, die mit einem Gewinde der Kolbenstange zusammenwirkt,

Fig. 4 einen Schnitt entlang der Linie IV-IV von Fig. 3

Fig. 5 eine Seitenansicht eines Druckinfusionsapparates mit einer an der Haltevorrichtung gelagerten Schnecke, die mit einer an dem dem Spritzenzylinder angeformten Gewindeschale zusammenwirkt und

Fig. 6 eine Stirnansicht der Vorrichtung nach Fig. 5 aus Richtung des Pfeiles VI.

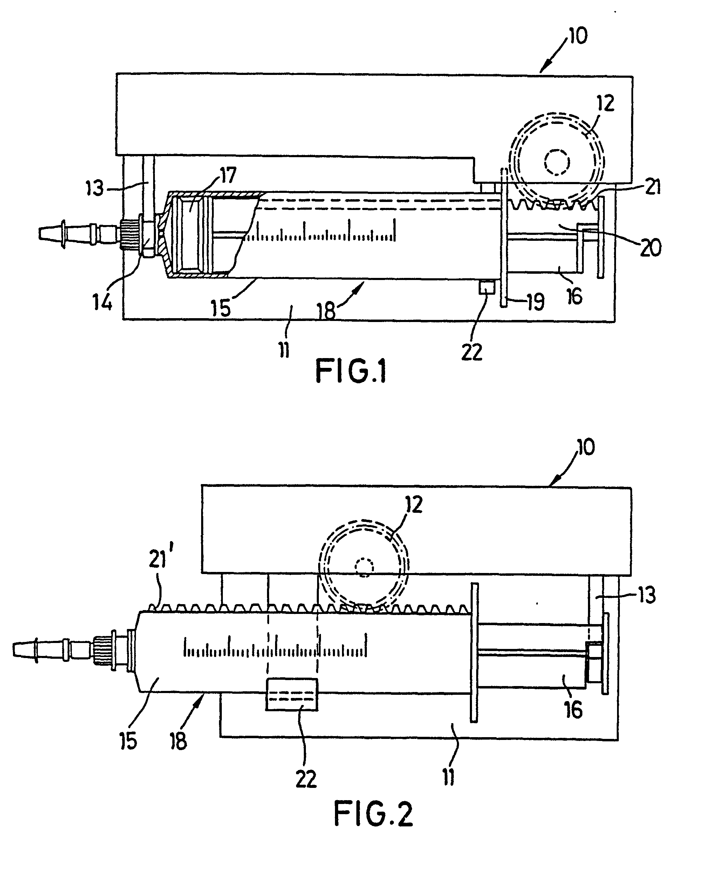

Der Druckinfusionsapparat nach Fig. 1 weist eine Haltevorrichtung 10 auf, die am oberen Ende eines Sockels 11 angebracht ist und seitlich von dem Sockel 11 absteht. Die Haltevorrichtung 10 enthält einen (nicht dargestellten) Antriebsmotor mit Untersetzungsgetriebe zum Antrieb eines Zahnrades 12. Das Zahnrad 12 ist mit horizontaler Achse angeordnet und ragt mit einem Teil seines Umfangs nach unten aus dem Gehäuse der Haltevorrichtung 10 heraus.

An dem vorderen Ende der Haltevorrichtung 10 befindet sich ein nach unten abstehender Halter 13, der eine seitliche Ausnehmung zum Einsetzen des Halses 14 des Spritzenzylinders 15 aufweist. Aus dem rückwärtigen Ende des Spritzenzylinders 15 ragt die Kolbenstange 16 heraus, die mit dem Kolben 17 im Innern des Spritzenzylinders 15 fest verbunden ist. Fig. 1 zeigt die aus dem Spritzenzylinder 15, dem Kolben 17 und der Kolbenstange 16 bestehende Spritze 18 im Endzustand der Druckinfusion, also bei vollständig ausgedrückter Spritze.

Die Kolbenstange 16 hat ein kreuzförmiges Profil, und die rückwärtige Begrenzungswand 19 des Spritzenzylinders 15 weist eine kreuzförmige Öffnung auf, durch die die Kolbenstange 18 passend hindurchragt. Auf diese Weise ist die Kolbenstange 18 relativ zu dem Spritzenzylinder 15 gegen Drehung gesichert.

Der nach oben weisende Steg 20 des Kreuzprofiles der Kolbenstange 16 ist mit einer zahnstangenartigen Verzahnung 21 versehen, die sich über die gesamte Länge der Kolbenstange 16 erstreckt. Wie Fig. 1 zeigt, ist die Länge der Kolbenstange 16 so bemessen, daß die Verzahnung 20 selbst bei voll eingeschobenem Kolben 17 noch ein Stück weit aus dem Spritzenzylinder 15 herausragt.

Die Verzahnung 21 der Kolbenstange 18 greift von unten her in die Verzahnung des Zahnrades 12 ein. Wenn das Zahnrad 12 gedreht wird, führt daher die Kolbenstange 16 eine Linearbewegung aus.

Zur besseren Abstützung des Spritzenzylinders 18 ist eine Abstützvorrichtung 22 vorgesehen, die von der Haltevorrichtung 10 nach unten absteht und eine seitliche Öffnung aufweist, in die das rückwärtige Ende des Spritzenzylinders eingeschoben werden kann.

Die Spritze 18 wird seitlich (gemäß Fig. 1 aus Richtung des Betrachters) in die seitlichen Öffnungen des Halters 13 und der Abstützvorrichtung 22 eingeschoben, wobei die Verzahnung 21 mit der Verzahnung des Zahnrades 12 in Eingriff kommt. Wird anschließend das Zahnrad 12 im Uhrzeigersinn angetrieben, dann wird der Kolben 17 im Innern des Spritzenzylinders 15 vorgeschoben.

Das Ausführungsbeispiel der Fig. 2 unterscheidet sich von demjenigen der Fig. 1 dadurch, daß das Spritzengehäuse 15 mit einer zahnstangenartigen längslaufenden Verzahnung 21' versehen ist, während das rückwärtige Ende der Kolbenstange 16 an einem von der Haltevorrichtung 10 nach unten abstehenden Halter 13 so festgelegt ist, daß die Kolbenstange 16 gegen axiale Verschiebungen und gegen Drehungen gesichert ist. Da andererseits die Kolbenstange 16 relativ zu dem Spritzenzylinder 15 drehfest ist, ist auch der Spritzenzylinder 15 gegen Drehung gesichert.

Der Spritzenzylinder 15 ist von einer Stützvorrichtung 22 abgestützt, die quer unterhalb des Spritzenzylinders 15 verläuft und auf der Spritzenzylinder 15 bei seiner Vorwärtsbewegung gleitet.

Bei dem Ausführungsbeispiel nach Fig. 2 wird bei einer Drehung des Zahnrades 12 im Gegenuhrzeigersinn der Spritzenzylinder 15 in Richtung auf den Halter 13 bewegt, wodurch die in der Spritze enthaltene Flüssigkeit ausgedrückt wird. Bei diesem Ausführungsbeispiel braucht die Kolbenstange 16 bei vollständig entleerter Spritze nicht wesentlich aus dem rückwärtigen Ende des Spritzenzylinders herauszuragen. Sie kann also kürzer sein als bei dem Ausführungsbeispiel der Fig. 1.

Anstelle der Verwendung des Zahnrades 12, das mit der zahnstangenartigen Verzahnung 21 bzw. 21' zusammenwirkt, kann auch die Kolbenstange oder der Spritzenzylinder ein Gewinde aufweisen, in das ein entsprechendes Gegengewinde eingreift, welches von dem in der Haltevorrichtung enthaltenen Antrieb angetrieben ist.

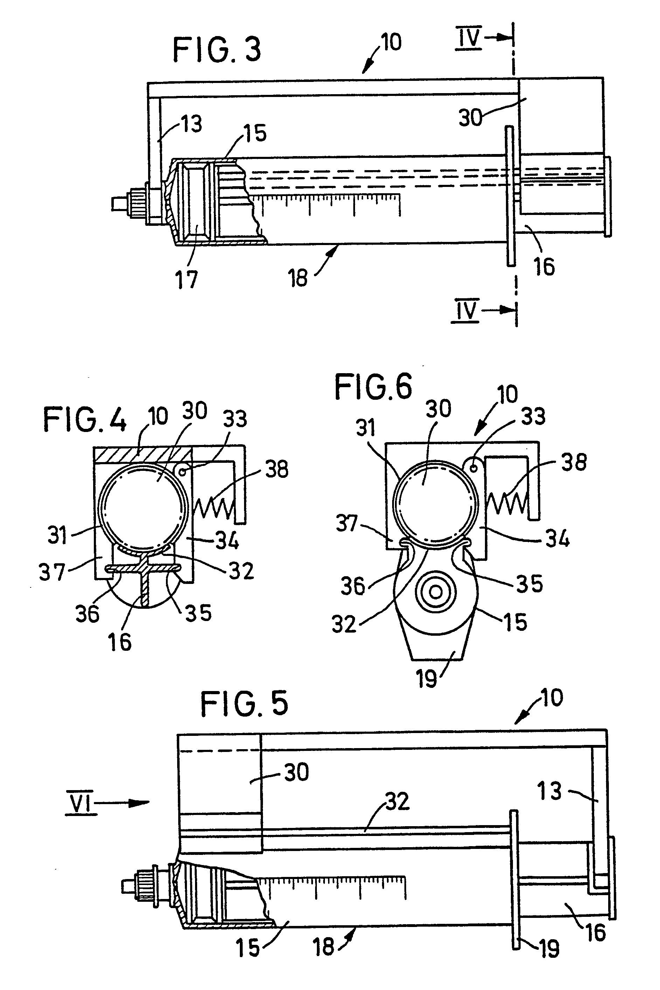

Derartige Druckinfusionsapparate sind in den Fig. 3 bis 6 dargestellt.

Bei dem Ausführungsbeispiel der Fig. 3 und 4 weist die Haltevorrichtung 10 einen Halter 13 zur Festlegung des vorderen Endes des Spritzenzylinders 15 auf. An der Haltevorrichtung 10 ist in (nicht dargestellten) Lagern ein Schneckenrad 30 gelagert, das drehend angetrieben ist. Das Schneckengewinde 31 am Umfang des Schneckenrades 30 greift mit dem Gegengewinde einer Gewindeschale 32 zusammen, die der Kolbenstange 18 einstückig angeformt ist. An der Haltevorrichtung 10 ist ein um eine horizontale Achse 33 schwenkbarer Hebel 34 angelenkt, der in der Nähe seines unteren Endes eine Ausnehmung 35 aufweist, welche das Ende eines horizontalen Steges der Kolbenstange 16 umgreift. Das andere Ende des horitzontalen Steges greift in eine Ausnehmung 36 eines mit der Haltevorrichtung 10 festverbundenen Halters 37 ein. Eine Feder 38 drückt den Hebel 34 in die Schließstellung, in der er parallel zu dem Halter 37 verläuft. Auf diese Weise wird die Kolbenstange 16 von dem Halter 37 und von dem Hebel 34 seitlich umschlossen und gegen seitliche Verschiebungen gesichert.

Wenn bei dem Ausführungsbeispiel der Fig. 3 und 4 das Schneckenrad 30 von einem Antriebsmotor angetrieben wird, wird die Kolbenstange 16 linear verschoben. Die Kolbenstange 16 bewegt sich also innerhalb der Ausnehmungen 35 und 36 linear. Zum Entfernen der Spritze 18 wird der Hebel 34 gegen die Wirkung der Feder 38 nach außen geschwenkt, so daß die Kolbenstange 16 freigegeben wird und nach unten abgezogen werden kann. Die Gewindeschale 32 mit dem Gegengewinde erstreckt sich über die gesamte Länge der Kolbenstange 16 und ist dem vertikalen Steg dieser Kolbenstange einstückig angeformt.

Bei dem Ausführungsbeispiel der Fig. 5 und 6 ist die Gewindeschale 32 der Außenseite des Spritzenzylinders 15 angeformt und erstreckt sich über dessen gesamte Länge. Der Halter 13 der Haltevorrichtung 10 greift an dem äusseren Ende der Kolbenstange 16 an und hält dieses fest. An dem dem Halter 13 abgewandten Ende der Haltevorrichtung 10 ist das Schneckenrad 30 gelagert. Dieses Schneckenrad 30 wird von einem (nicht dargestellten) Antriebsmotor um eine parallel zu dem Spritzengehäuse verlaufende Achse gedreht. Das Gewinde 31 am Umfang des Schneckenrades 30 greift in das Gegengewinde der langgestreckten Gewindeschale 32 des Spritzenzylinders 15 ein.

Um die beiden Gewindeteile 31,32 in gegenseitigem Eingriff zu halten, ist an der Haltevorrichtung 10 ein fester Halter 37 und ein schwenkbarer Hebel 34 angeordnet. Der Halter 37 und der Hebel 34 weisen Ausnehmungen 36 bzw. 35 auf, die parallele Wülste des Spritzenzylinders 15 umgreifen.

Wenn bei dem Ausführungsbeispiel der Fig. 5 und 6 das Schneckenrad 30 gedreht wird, wird der Spritzenzylinder 15 relativ zu der festgehaltenen Kolbenstange 16 linear bewegt. Dabei gleitet der Spritzenzylinder 15 mit den Wülsten in den Ausnehmungen 35 und 36. Zum Entfernen der Spritze 18 von der Haltevorrichtung 10 wird der Hebel 34 entgegen der Wirkung der Feder 38 nach außen geschwenkt, so daß die Ausnehmungen 35 und.36 die Spritze freigeben.

## Patentansprüche

1. Druckinfusionsapparat für medizinische Anwendungen, mit einer Haltevorrichtung (10), einer an der Haltevorrichtung lösbar befestigten Spritze (15, 16) und einem an der Haltevorrichtung bewegbar angebrachten Antriebsorgan zum Bewegen der Kolbenstange (16) der Spritze relativ zu dem Spritzenzylinder (15), wobei das Antriebsorgan (12) drehend angetrieben ist und mit einer die Drehung in eine Linearbewegung umsetzenden Profilierung (21; 21'; 31) zum Antrieb der Kolbenstange (16) oder des Spritzenzylinders (15) zusammenwirkt, dadurch gekennzeichnet, daß die Profilierung (21; 21'; 31) an der Kolbenstange (16) oder an dem Spritzenzylinder der als Einmalspritze ausgebildeten Spritze vorgesehen ist und daß die Profilierung (21; 21'; 31) beim Einsetzen der Spritze in die Haltevorrichtung (10) mit dem drehbaren Antriebsorgan in Eingriff kommt.

2. Druckinfusionsapparat nach Anspruch 1, dadurch gekennzeichnet, daß das Antriebsorgan ein Zahnrad (12) ist, dessen Zähne in eine zahnstangenartige Verzahnung (21; 21') der Kolbenstange (16) oder des Spritzenzylinders (15) eingreifen.

3. Druckinfusionsapparat nach Anspruch 1, dadurch gekennzeichnet, daß das Antriebsorgan (30) ein Gewinde (31) aufweist, das mit einem Gegengewinde (32) der Kolbenstange (16) oder des Spritzenzylinders (15) zusammenwirkt.

4. Druckinfusionsapparat nach einem der Ansprüche 1 bis 3, dadurch gekennzeichnet, daß ein den Spritzenzylinder (15) in axialer Richtung festlegender und gegen Drehung sichernder Halter (13) vorgesehen ist und daß das rotierende Antriebsorgan (12; 30) die Kolbenstange (16) linear bewegt.

5. Druckinfusionsapparat nach Anspruch 4, dadurch gekennzeichnet, daß der Spritzenzylinder von unten durch eine Stützvorrichtung (22; 34; 37) abgestützt ist.

## Claims

1. Pressure infusion apparatus for medical uses, comprising a holding means (10), a syringe (15, 16) detachably secured to the holding means and a driving member movably mounted at the holding means for moving the piston rod (16) of the syringe relative to the syringe cylinder (15), the driving member (12) being driven rotatingly and coacting with a profile (21; 21'; 31) converting the rotation into a linear movement, for driving the piston rod (16) or the syringe cylinder (15),

characterized in that the profile (21; 21'; 31) is provided at the piston rod (16) or at the syringe cylinder of the syringe of the single-use type, and that the profile (21; 21'; 31) eengages the rotatable driving member when the syringe is mounted in the holding means (10).

2. Pressure infusion apparatus as defined in claim 1, characterized in that the driving member is a toothed wheel (12) whose teeth engage a rack-type toothing (21; 21') of the piston rod (16) or of the syringe cylinder (15).

3. Pressure infusion apparatus as defined in claim 1, characterized in that the driving member (30) has a thread (31) which cooperates with a counterthread (32) of the piston rod (16) or of the syringe cylinder (15).

4. Pressure infusion apparatus as defined in one of claims 1 to 3, characterized in that a holder (13) is provided to retain the syringe cylinder (15) in axial direction and to secure it against rotation and that by the rotating driving member (12; 30), the piston rod (16) is moved linearly.

5. Pressure infusion apparatus as defined in claim 4, characterized in that the syringe cylinder is backed from below by a supporting means (22; 34; 37).

## Revendications

1. Appareil de perfusion sous pression pour applications médicales, comportant un dispositif de retenue (10), une seringue (15, 16) fixée de façon détachable au dispositif de retenue, et un organe d'entraînement monté de manière à être mobile sur le dispositif de retenue et servant à déplacer la tige de piston (16) de la seringue par rapport au cylindre (15) de la seringue, l'organe d'entraînement (12) étant entraîné en rotation et coopérant avec un profil (21; 21'; 31) qui convertit le mouvement de rotation en un déplacement linéaire et est utilisé pour l'entraînement de la tige de piston (16) ou du cylindre (15) de la seringue, caractérisé en ce que le profil (21; 21'; 31) est prévu sur la tige de piston (16) ou sur le cylindre de la seringue réalisée sous la forme d'une seringue à usage unique, et en ce que le profil (21; 21'; 31) engrène avec l'organe d'entraînement rotatif lors de l'insertion de la seringue dans le dispositif de retenue (10).

2. Appareil de perfusion sous pression selon la revendication 1, caractérisé en ce que l'organe d'entraînement est un pignon (12), dont les dents engrènent dans une denture (21; 21'), en forme de crémaillère, de la tige de piston (16) ou du cylindre (15) de la seringue.

3. Appareil de perfusion sous pression selon la revendication 1, caractérisé en ce que l'organe d'entraînement (30) comporte un filetage (31) qui coopère avec un filetage antagoniste (32) de la tige de piston (16) ou du cylindre (15) de la seringue.

4. Appareil de perfusion sous pression selon l'une des revendications 1 à 3, caractérisé en ce qu'il est prévu un support (13), qui fixe le cylindre (15) de la seringue dans la direction axiale et le bloque contre toute rotation, et en ce que l'organe d'entraînement rotatif (12; 30) déplace linéairement la tige de piston (16).

5. Appareil de perfusion sous pression selon la revendication 4, caractérisé en ce que le cylindre de la seringue est soutenu par en-dessous, par un dispositif de soutien (22; 34; 37).

FIG.1

FIG.2

0 092 712

FIG. 3

FIG.4  FIG.6

FIG. 5

3